# EUROPÄISCHE PATENTSCHRIFT

(11) **EP 4 210 599 B1**
(45) Veröffentlichungstag und Bekanntmachung des Hinweises auf die Patenterteilung: **11.12.2024**
(21) Anmeldenummer: 21735641.9
(22) Anmeldetag: 21.06.2021
(51) Int. Cl.: A61B 17/132

(54) **ABSCHNÜRVORRICHTUNG FÜR KÖRPERTEILE**
LIGATURE DEVICE FOR BODY PARTS
DISPOSITIF DE LIGATURE POUR PARTIES DE CORPS

(30) Priorität: 09.09.2020 DE 102020123496
(43) Veröffentlichungstag der Anmeldung: 19.07.2023
(73) Patentinhaber: Kimetec GmbH, 71254 Ditzingen (DE)
(72) Erfinder: KIRCHNER, Claudia, 71706 Markgröningen (DE); KIRCHNER, Hansjörg, 71706 Markgröningen (DE); IHLE, Caroline, 71706 Markgröningen (DE)
(74) Vertreter: Fleck, Hermann-Josef
(86) Internationale Anmeldenummer: PCT/EP2021/066864
(87) Internationale Veröffentlichungsnummer: WO 2022/053193

(56) Entgegenhaltungen:
- EP-A2- 0 566 036
- EP-B1- 1 458 296
- WO-A2-2019/057784
- US-A- 5 451 234
- US-A1- 2011 247 179
- US-A1- 2012 296 369
- US-A1- 2017 273 694
- US-B1- 6 250 047

## Beschreibung

Die Erfindung bezieht sich auf eine Abschnürvorrichtung für Körperteile, insbesondere Venenstauer, mit einem Schlossgehäuse, in dem ein unten von einer Bodenwand, seitlich von zwei Seitenwänden und oben von einer Deckwand umgrenzter, in Längsrichtung verlaufender Durchführschacht gebildet ist, und mit einem Abschnürband, das mittels eines an seinem einen, dem rückseitigen Ende befestigten Rastschuh lösbar an einem hinteren Abschnitt des Schlossgehäuses verrastet oder verrastbar und mit seinem anderen Ende zum Bilden einer Schlaufe variierbarer Größe von der Rückseite her durch den Durchführschacht geführt oder führbar und entsprechend der Größe eines aufgenommenen Körperteils mittels eines Klemmmittels, insbesondere mittels einer in dem Schlossgehäuse in einer Schwenklagerung schwenkbar gelagerten Wippe, festklemmbar ist.

Eine Abschnürvorrichtung dieser Art ist in der EP 1 458 296 B1 angegeben. Bei dieser bekannten Abschnürvorrichtung für Körperteile ist in einem Schlossgehäuse, das eine Bodenwand, zwei Seitenwände und auf seiner Oberseite eine Deckwand aufweist, ein auf der Rückseite und der Vorderseite des Schlossgehäuses offener Durchführschacht für ein Abschnürband gebildet. Das Abschnürband ist an seinem hinteren Ende mit einem Rastschuh versehen und über diesen in einem hinteren Abschnitt des Schlossgehäuses lösbar verrastbar und mit seinem vorderen Ende von der Rückseite des Schlossgehäuses her unter Bildung einer Schlaufe variierbarer Größe durch den Durchführschacht geführt, so dass ein abzuschnürendes Körperteil in der Schlaufe aufnehmbar und mit einer erforderlichen Spannung abschnürbar ist, um z. B. einen venösen Blutfluss zu unterbinden. Das Abschnürband wird unter dem von einer Bedienperson eingestellten Spannungszustand in dem Schlossgehäuses mittels einer Wippe gegen eine in dem Schlossgehäuse angeformte Zwischenwand festgeklemmt und kann zum Aufheben des Spannungszustands durch Betätigen der Wippe unter Vergrößerung der Schlaufe freigegeben werden. Durch Entrasten des Rastschuhs kann dieser aus dem Schlossgehäuse gezogen und die Schlaufe vollständig geöffnet werden. Sowohl aus Sicht der zu behandelnden Person als auch des Benutzers, der sich auf die eigentlichen Behandlungsmaßnahmen zu konzentrieren hat, kommt einer möglichst guten Funktionsfähigkeit der Abschnürvorrichtung, zu der auch hohe hygienische Anforderungen zu rechnen sind, wesentliche Bedeutung zu.

Eine vom Grundaufbau her ähnliche Abschnürvorrichtung wie die vorstehend genannte ist auch in der EP 0 633 747 B1 gezeigt.

Die WO 2019/057784 A2 zeigt eine Abschnürvorrichtung zum Stauen von Gefäßen mit einem Stauband, welches mit Hilfe eines Verschlusses zu einem Rundband verbunden wird. Das Stauband besteht aus einem textilfreien Material und das Stauband und/oder der Verschluss sind mit einer Gleitbeschichtung versehen, die insbesondere aus einer Fluorierung der Oberfläche besteht.

Eine insbesondere auch für einen einmaligen Gebrauch geeignete, kostengünstiger und einfacher herzustellende Abschnürvorrichtung, insbesondere in Ausführung als Venenstauer, ist in der EP 3 068 314 B1 vorgestellt. Das Abschnürband ist dabei mit einer einfachen Verriegelungsvorrichtung, insbesondere einem Klettverschluss, versehen und besteht dazu aus einem flauschigen Material, welches Schlaufen für die Klettverriegelung bildet und z. B. ein Schlinggewebe oder ein Spinnvlies umfasst, also aus Fäden oder Härchen zusammengesetzt ist, wobei als geeignete Materialien Viskose, Polyester oder Polypropylen genannt sind. Das Material ist flexibel und kann eine Eigenelastizität aufweisen.

In der DE 44 12 832 A1 ist eine Autoklavierbare Manschette gezeigt, die als Fourniquet-Manschette oder als Blutdruck-Manschette ausgebildet sein kann. Dabei ist auf einer flachen Unterlage eine als langgestreckter Schlauch ausgebildete Seele aufgeklebt, wobei die Unterlage und die Seele aus einem durch Dampfsterilisation sterilisierbaren Material bestehen, beispielsweise aus Silikon. Insbesondere besteht die Unterlage aus härterem Silikon-Material als die Seele. Ein solcher mehrschichtiger Aufbau des Abschnürbands ist für einen Venenstauer mit möglichst kompaktem, einfach mit einer Hand bedienbaren Schlossgehäuse mit Nachteilen behaftet.

Bei einer in der EP 0 566 036 A2 offenbarten Abschnürvorrichtung für Körperteile, insbesondere Venenstauer, ist ein Abschnürband durch ein Schlossgehäuse geführt und wird darin in seinem an einem Körperteil angelegten, gespannten Zustand mittels einer um eine Achse schwenkbar gelagerten Wippe gegen eine Gehäusewand festgeklemmt. Die Wippe ist auf der im Gehäuse fest gehaltenen Achse nicht nur schwenkbar gelagert, sondern auch in Längsrichtung begrenzt verschieblich geführt. Eine Feder, die sich an der Achse abstützt, wirkt auf einen abgekröpften Schenkel der Wippe und sucht diese in Öffnungsstellung zu drücken. Beim Anlegen der Abschnürrvorrichtung wird die Wippe infolge der Abschnürzugkraft im Abschnürband gegen die Kraft der Feder in ihre der Öffnungsstellung entgegengesetzte Lage gezogen. Zum Lösen und Öffnen des Abschnürbands wird die Wippe mittels eines manuellen Auslösedrucks auf eine Drucktaste, die gleichzeitig auch einen Teil eines Rastverschlusses bildet, aus ihrer Klemmstellung in eine Lösestellung geschwenkt. Nach vorübergehender Unterbrechung des manuellen Auslösedrucks auf die Drucktaste kann die Feder die Wippe weiter in die Öffnungsstellung verschieben, wonach der Rastverschluss und damit die durch das Abschnürband gebildete Schlaufe geöffnet werden kann. Das Schlossgehäuse besitzt dabei filigran auf diesen besonderen Funktionsmechanismus abgestimmte Ausgestaltungsmerkmale, wobei sowohl das Lösen der Abschnürung als auch das Öffnen der Schlaufe durch manuelle Betätigung über die Drucktaste erfolgen. Die Feder dient dabei zum Verschieben der Wippe; deren Verschwenken erfolgt zum Klemmen über das Abschnürband und zum Lösen sowie Öffnen über die Drucktaste. Über den Aufbau des Abschnürbands sind keine näheren Ausführungen gemacht.

Die US 6 250 047 B1 zeigt ein Einweg-Abschnürband für Blutgefäße, das ein thermoplastisches Elastomer mit Additiven aufweist, die Gleit- und Antiblockierkomponenten umfassen.

In der US 2011/247179 A1 ist ein Verschlussmechanismus zum Einklemmen eines Bandes gezeigt, das z. B. zum Abbinden um den Arm oder das Bein eines Patienten legbar ist.

Der vorliegenden Erfindung liegt die Aufgabe zugrunde, eine Abschnürvorrichtung für Körperteile, insbesondere in Ausführung als Venenstauer, der eingangs genannten Art bereitzustellen, die sowohl aus Sicht der zu behandelnden Person als auch des Benutzers optimierte Funktionseigenschaften besitzt.

Diese Aufgabe wird bei einer Abschnürvorrichtung mit den Merkmalen des Anspruchs 1 gelöst. Hierbei ist bei einer Abschnürvorrichtung, insbesondere einem Venenstauer, mit den im Oberbegriff genannten Merkmalen vorgesehen, dass das Abschnürband aus elastischem Material, wie einem Naturgummi, thermoplastischem Elastomer (TPE) oder insbesondere aus Silikon, also als Silikonband, hergestellt ist. Wie die Erfinder herausgefunden haben, hat das so gummielastisch ausgebildete Abschnürband, insbesondere einstückige, vorzugsweise als Vollprofil ausgebildete Silikonband, sowohl für die Handhabung im Zusammenwirken mit dem Schlossgehäuse als auch aufgrund seiner Anschmiegbarkeit um die Haut des abzuschnürenden Körperteils und einer gewissen Elastizität zum Dosieren der Abschnürspannung und zudem aufgrund seiner hygienischen Eigenschaften für die Funktionalität der Abschnürvorrichtung wesentliche Vorteile. Das Abschnürband ist zum einen aufgrund seiner guten Gleiteigenschaften (geringe Reibung) im unbelasteten Zustand im Schlossgehäuse und in der Hand gut führbar und zum anderen im Durchführschacht des Schlossgehäuses auch aufgrund seiner gummielastischen Konsistenz mittels der in das Bandmaterial eingreifenden Wippe und/oder Gehäusestruktur gut festklemmbar. Die einstückige Ausbildung, insbesondere als Vollprofil, hat Vorteile für den Aufbau, die Funktion und Handhabung.

Eine gute Griffigkeit bei gleichzeitig guten Gleiteigenschaften (geringe Reibungseinflüsse) wird dabei dadurch begünstigt, dass die Bandoberseite und/oder die Bandunterseite mit einer reibungsmindernden bzw. reibungsarmen reliefförmigen Struktur versehen ist. Die guten Gleiteigenschaften werden dabei unter Verminderung der Kontaktflächen bzw. Reibflächen zwischen dem Abschnürband und zugewandten Flächenbereichen im Schlossgehäuse erhalten bzw. beibehalten. Die Griffigkeit und Gleitfähigkeit bzw. geringe Reibung (die mehr mit der Mikrostruktur der sich kontaktierenden Oberflächenbereiche zusammenhängt) können dabei durch Versuche empirisch optimiert werden.

Ist vorgesehen, dass das Abschnürband eine geglättete und/oder gehärtete Oberfläche aufweist, werden verbesserte Gleiteigenschaften sowohl auf der Haut der zu behandelnden Person als auch beim Verschieben im Schlossgehäuse erreicht, da die Reibung (insbesondere Haftreibung, aber auch Gleitreibung) vermindert wird. Die Glättung der Oberfläche und/oder Härtung des Abschnürbands, insbesondere Silikonbands oder TPE-Bands, lässt sich dabei günstig durch Einbringen von diese Eigenschaften bestimmenden Zusätzen beeinflussen oder z.B. auch durch Behandlung mit UV-Strahlung. Dabei ist auch eine Abstimmung auf das Material und Strukturen des Schlossgehäuses möglich, wobei eine Optimierung durch praktische Versuche oder Simulation erfolgen kann.

Eine vorteilhafte Maßnahme zum Erzielen einer geglätteten und gehärteten Oberfläche besteht z. B. darin, dass die Oberflächenglättung und -härtung durch Fluoridbehandlung eines gummielastischen Bands, insbesondere Silikonbands, vorgenommen ist, aus dem das Abschnürband hergestellt ist.

Für die Herstellung und die Funktion sind des Weiteren die Maßnahmen von Vorteil, dass das (insbesondere einstückige, vorzugsweise als Vollprofil ausgebildete) Abschnürband einen rechteckförmigen Querschnitt aufweist, wobei die Breite des Abschnürbands zwischen 10 und 40 mm, vorzugsweise zwischen 20 und 30 mm, und die Dicke des Abschnürbands zwischen 0,8 und 3 mm, vorzugsweise zwischen 1 und 2,5 mm, beträgt.

Damit die Bedienperson den über die Schlaufe des Abschnürbands an dem abzuschnürenden Körperteil ausgeübten Spannungszustand auf einfache Weise kontrollieren kann, ist vorteilhaft vorgesehen, dass das Abschnürband mit einer sich bei seiner Dehnung ändernden Markierung versehen ist, anhand deren ein einer Abschnürspannung entsprechender Spannungszustand des Abschnürbands erkennbar ist.

Zum Erfüllen hygienischer Erfordernisse kann vorteilhaft vorgesehen sein, dass das Abschnürband transparent ist, insbesondere auch für Strahlung im UV-Bereich, wobei insbesondere eine Transparenz im UVC-Bereich vor allem aus hygienischen Gründen von Vorteil ist.

Entsprechend tragen auch die Maßnahmen, dass das Schlossgehäuse transparent für UV-Strahlung, insbesondere UVC-Strahlung, ist, zu einer vorteilhaften hygienischen Behandlung der Abschnürvorrichtung bei, da schädliche Keime, wie Viren, Bakterien, Pilze und weitere Schadstoffe auch in mit Reinigungsflüssigkeit bzw. mechanischen Reinigungsmitteln schlecht zugänglichen Stellen im Schlossgehäuse durch die UV-Strahlung erreicht und unschädlich gemacht werden.

Zudem kann die UV-Strahlung zum Bewirken einer absichtlichen Alterung, insbesondere in Folge einer erkennbaren Änderung der Transparenz oder einer Farbwirkung genutzt werden, um bereits ältere Abschnürvorrichtungen auszusondern.

Zum Erreichen günstiger Gleiteigenschaften des Abschnürbands in dem Durchführschacht des Schlossgehäuses ist des Weiteren vorteilhaft vorgesehen, dass in dem Durchführschacht an mindestens einer der Bandoberseite und/oder der Bandunterseite zugekehrten Seite von Wandabschnitten oder einer gegebenenfalls vorhandenen Wippe vortretende, in Längsrichtung des Schlossgehäuses verlaufende rippenartige Gleitstrukturen angeordnet sind. Dadurch werden große, ungünstige Reibflächen zwischen dem Abschnürband und zugekehrten Wirkflächen in dem Schlossgehäuse vermieden.

Für die Funktion und Handhabung sind des Weiteren die Maßnahmen von Vorteil, dass die Wippe auf ihrer Unterseite in ihrem zur Rückseite hin hinter der Schwenklagerung gelegenen rückseitigen Abschnitt gegen die Oberseite der Bodenwand mittels einer Federanordnung abgestützt ist, durch die die Wippe bei unbelasteter Schlaufe in ihre nicht klemmende Ruhelage gebracht ist. Hierdurch kann das Abschnürband im aufgehobenen Spannungszustand leicht in dem Schlossgehäuse verschoben werden, um z. B. eine geeignete Schlaufengröße voreinzustellen oder das Schlossgehäuse relativ zu dem Abschnürband z. B. zum Anlegen, Abnehmen oder Aufbewahren in eine gewünschte Position zu bringen.

Eine vorteilhafte Ausgestaltung besteht dabei darin, dass die Federanordnung als an der Wippe angeformte, zur Bodenwand hin vorstehende Rückstellfeder ausgebildet ist.

Vorteile für die Handhabung, wie z. B. Aufbewahrung, ergeben sich ferner dadurch, dass das Abschnürband an seinem von dem Rastschuh abgewandten anderen, dem vorderseitigen Ende mit einem als Halterung ausgebildeten Abschlussstück versehen ist.

Die Erfindung wird nachfolgend anhand von Ausführungsbeispielen unter Bezugnahme auf die Zeichnungen näher erläutert. Es zeigen:
- Fig. 1: eine Abschnürvorrichtung mit einem durch ein Schlossgehäuse geführten und eine Schlaufe bildenden Abschnürband in perspektivischer Darstellung,
- Fig. 2A: eine Draufsicht der Abschnürvorrichtung nach Fig. 1,
- Fig. 2B: eine geschnittene seitliche Ansicht der Abschnürvorrichtung entlang einer Schnittlinie A-A nach Fig. 2A,
- Fig. 2C: einen vergrößerten Ausschnitt Z der Abschnürvorrichtung nach Fig. 2B im Bereich des Schlossgehäuses,
- Fig. 3A: eine perspektivische Ansicht des Schlossgehäuses von der Rückseite, schräg von unten,
- Fig. 3B: das Schlossgehäuse von der Rückseite,
- Fig. 3C: das Schlossgehäuse von oben,
- Fig. 4A: eine in das Schlossgehäuse zum Festklemmen des Abschnürbands einsetzbare Wippe in Draufsicht,
- Fig. 4B: die Wippe nach Fig. 4A in perspektivischer Ansicht schräg von vorne oben,
- Fig. 4C: die Wippe nach Fig. 4A in perspektivischer Ansicht schräg von vorne unten,
- Fig. 4D: einen Querschnitt der Wippe nach Fig. 4A entlang einer Schnittlinie B-B,
- Fig. 4E: eine Vorderansicht der Wippe nach Fig. 4A und
- Fig. 4F: einen Längsschnitt der Wippe entlang einer Schnittlinie A-A nach Fig. 4E.

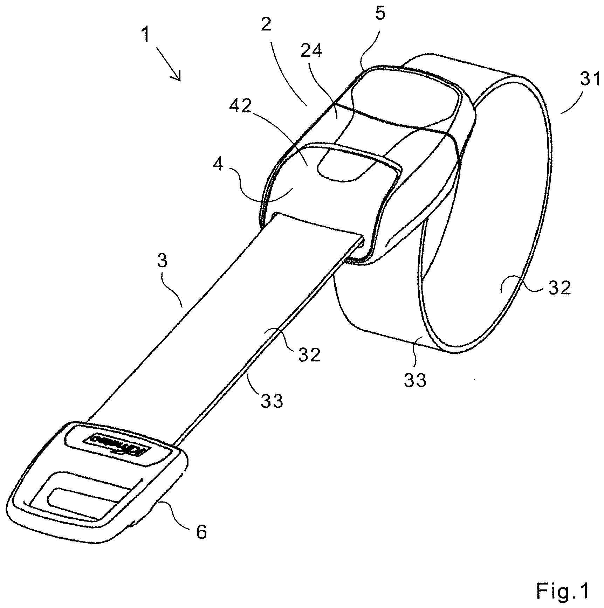

Fig. 1 zeigt in perspektivischer Darstellung schräg von vorn oben ein Ausführungsbeispiel für eine Abschnürvorrichtung 1 mit ihren wesentlichen Bestandteilen, nämlich einem Schlossgehäuse 2, einer in diesem schwenkbar gelagerten Wippe 4 und einem Abschnürband 3, das in Längsrichtung durch einen auf seiner Rückseite und seiner Vorderseite offenen Durchführschacht im Innern des Schlossgehäuses 2 geführt und unter Bildung einer Schlaufe 31 variierbarer Größe mit einem Rastschuh 5 in einem rückseitigen Abschnitt des Schlossgehäuses 2 lösbar verrastet ist. Das Abschnürband 3 ist mit seinem rückseitigen (hinteren) einen Ende bzw. Endabschnitt an dem Rastschuh befestigt und unter Bildung der Schlaufe 31 von der Rückseite des Schlossgehäuses 2 her durch den auf seiner Rückseite offenen Durchführschacht oberhalb einer (in Fig. 1 nicht ersichtlichen) Wippenplatte einer Wippe 4 in Längsrichtung durch das Schlossgehäuse 2 und durch eine in der Vorderseite der Wippe 4 eingebrachte Durchführöffnung 43 aus dem Schlossgehäuse 2 herausgeführt. An seinem anderen, vorderen Ende bzw. Endabschnitt ist das Abschnürband 3 mit einem als Halterung dienenden Abschlussstück 6 versehen. Gemäß Fig. 1 weist das Schlossgehäuse 2 auf seiner Oberseite eine Deckwand 24, seitlich auf jeder Seite eine Seitenwand (von denen in Fig. 1 nur eine zu sehen ist) und eine (in Fig. 1 nur wenig ersichtliche) Bodenwand auf, die den Durchführschacht oben, seitlich und unten umgeben.

Mittels der schwenkbar in einer Schwenklagerung in dem Schlossgehäuse 2 gelagerten Wippe 4 kann das Abschnürband 3 durch Zug an dem nach Fig. 1 der Rückseite des Schlossgehäuses 2 benachbarten Abschnitt der Schlaufe 31 (mit einer Kraftkomponente) nach unten festgeklemmt werden. Durch den Zug des von der Schlaufe 31 in den Durchführschacht übergehenden Abschnitts des Abschnürbands 3 wird der hinter der Schwenklagerung liegende Abschnitt der Wippe 4 nach unten und der vor der Schwenklagerung gelegene Abschnitt der Wippe 4 nach oben geschwenkt und das über die Wippe 4 verlaufende Abschnürband 3 durch den vorderen Teil der Wippenplatte gegen ein oberhalb des Abschnürbands 3 in dem Schlossgehäuse 2 fest angebrachtes (in Fig. 1 nicht ersichtliches) Widerlager gedrückt und dadurch festgeklemmt. Dadurch kann eine zum Abschnüren eines in der Schlaufe aufgenommenen Körperteils erforderliche Schlaufenweite fixierbar eingestellt werden, da durch einen Zug an dem auf der Vorderseite des Schlossgehäuses 2 herausgeführten Abschnitt des Abschnürbands 3 das abzuschnürende Körperteil selbst den auf der Rückseite des Schlossgehäuses 2 über der Wippe 4 einlaufenden Abschnitt des Abschnürbands 3 nach unten zieht.

Der vordere Abschnitt der Wippe 4 ist mit einem an der Wippenplatte 41 angeformten Betätigungsabschnitt 42 versehen, der von dem vorne liegenden Übergangsbereich zwischen der Wippenplatte und dem Betätigungsabschnitt 42 unter konvexer Wölbung nach oben hinten in eine im vorderen Bereich der Deckwand 24 des Schlossgehäuses 2 eingeformte Ausnehmung mit entsprechender Kontur hineinragt. Durch Druck auf den Betätigungsabschnitt 42 kann das mittels des vor der Schwenklagerung liegenden vorderen Abschnitts der Wippenplatte mit seiner Oberseite 32 gegen das Widerlager des Schlossgehäuses 2 gedrückte und dadurch festgeklemmte Abschnürband 3 dosiert gelöst bzw. eingeklemmt werden. Dadurch ist die Schlaufe 31 in ihrer Weite (Durchmesser bzw. Umfang) variierbar und dadurch das Abschnürband 3 unter gewünschter Abschnürspannung in einem entsprechenden Spannungszustand an dem abzuschnürenden Körperteil festlegbar. Durch Entrasten des Rastschuhs 5 kann die Schlaufe 31 vollständig geöffnet und somit die Abschnürvorrichtung 1 von dem abzuschnürenden Körperteil abgenommen werden und umgekehrt, durch Verrasten des Rastschuhs 5 in dem Schlossgehäuse 2 bei entsprechend vorgewählter Schlaufengröße um ein abzuschnürendes Körperteil angelegt werden. Dabei bildet die Bandoberseite 33 des Abschnürbands 3 die dem Körperteil zugekehrte Innenseite der Schlaufe 31. Dieser Aufbau und diese Funktion sind auch in der eingangs genannten EP 1 458 296 B1 gezeigt.

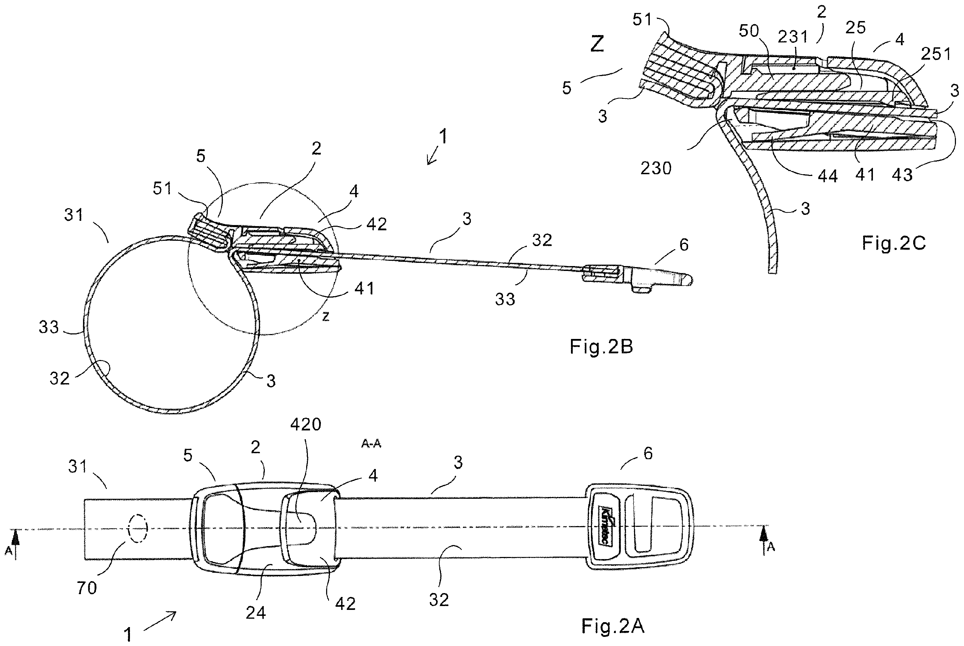

In den weiteren Fig. 2A, 2B, 2C; 3A, 3B; 4A, 4B, 4C, 4D, 4E und 4F sind die wesentlichen Bestandteile der Abschnürvorrichtung 1 und auch die Unterschiede gegenüber der in der EP 1 458 296 B1 vorgestellten Abschnürvorrichtung 1 in ihren Einzelheiten näher gezeigt.

Im Unterschied zu der in der EP 1 458 296 B1 und auch in der EP 0 633 747 B1 gezeigten Ausführung ist bei der in Fig. 1 und den Fig. 2A, 2B und 2C gezeigten Abschnürvorrichtung gemäß vorliegender Erfindung das Abschnürband 3 (insbesondere einstückig und dabei vorzugsweise als Vollprofil) aus einem elastischen Material, wie Naturgummi, einem thermoplastischen Elastomer (TPE) oder besonders bevorzugt aus Silikon als (einstückiges) Silikonband gefertigt. Das so ausgebildete Abschnürband, insbesondere Silikonband, besitzt von Hause aus eine bei üblicher Bandbreite der Abschnürvorrichtung 1 zwischen z. B. 10 und 40 mm, vorzugsweise zwischen 20 und 30 mm, und einer Banddicke zwischen z. B. 0,8 und 3 mm, vorzugsweise zwischen 1 und 2,5 mm, wie insbesondere in Ausbildung als Venenstauer zweckmäßig, eine für die Funktion vorteilhafte Flexibilität und auch Elastizität, insbesondere Gummielastizität. Dadurch kann sich das Abschnürband 3 vorteilhaft an unterschiedliche Umfangskonturen und Gewebeeigenschaften der Haut und Hautoberfläche in seiner Form gut anpassen und weist vornehmlich als Silikonband auch eine gute Schmiegsamkeit bzw. gute haptische Eigenschaften auf.

Um ein Gleiten auf der Haut und auch zwischen zugekehrten Wandbereichen innerhalb des Schlossgehäuses 2 und der Wippe 4 zu verbessern, ist das als Silikonband bzw. aus dem gummielastischen Material hergestellte Abschnürband 3 insgesamt oder nur auf seiner Oberseite 32 und/oder Unterseite 33 durch chemische, mechanische bzw. physikalische Behandlung geglättet und/oder (gegenüber seinem Innenbereich) gehärtet, beispielsweise durch Zugabe von die Gleiteigenschaften und/oder die Härte beeinflussenden Zusatzstoffen bei der Herstellung oder durch Behandlung mittels UV-Strahlung. Beispielsweise besteht diese Behandlung in einer Fluoridbehandlung. Die Bandoberseite 32 und/oder Bandunterseite 33 kann zusätzlich mit die Griffigkeit verbessernden reliefartigen, eingeformten Strukturen versehen sein, die sowohl für die Handhabung durch den Benutzer als auch für das Anfühlen durch die zu behandelnde Person Vorteile in der Haptik bieten und zudem für das Gleiten des Abschnürbands 3 in dem Schlossgehäuse 2 und sein Festklemmen in dem Schlossgehäuse 2 Vorteile ergibt. Das verbesserte Gleiten (im unbelasteten oder wenig belasteten Zustand des Abschnürbands) kann dabei durch die verringerte Kontaktfläche bzw. Reibefläche und durch die Oberflächenstruktur zwischen dem Abschnürband und zugekehrten Flächenbereichen im Inneren des Schlossgehäuses beeinflusst werden. Dabei sind die Bandoberseite 32 und die Bandunterseite 33 in reliefstrukturierter Ausführung hinsichtlich einer guten, einfachen Reinigbarkeit gestaltet, um hohe hygienische Anforderungen zu erfüllen.

Vorteilhaft kann das Abschnürband 3 mit einer Markierung 70 versehen sein, damit der Benutzer den Spannungszustand der an einem abzuschnürenden Körperteil angelegten Schlaufe 31 einfach kontrollieren kann. Beispielsweise besteht die Markierung 70 aus einem auf der dem Benutzer zugekehrten Bandoberseite 32 eingeprägten oder aufgedruckten geometrischen Muster, wie z. B. einer Ellipse mit quer (rechtwinklig) zur Längsrichtung des Abschnürbands 3 verlaufendem großen Durchmesser, die sich bei erforderlicher Abschnürspannung und dadurch gegebenen Spannungszustand der Schlaufe 31 in Folge der Elastizität des Abschnürbands 3 zu einem gut erkennbaren Kreis verformt. Auch andere geometrische Muster mit leicht erkennbarer, definierter Markierungsänderung (wie z. B. Rechteck mit Änderung in ein Quadrat) sind geeignet. Beispielsweise entspricht ein solcher erreichter Spannungszustand bei einem Venenstauer einer Abschnürspannung, die einem zum Unterbinden des venösen Blutflusses erforderlichen Druck zwischen 5 mm Hg und 40 mm Hg, beispielsweise 10 mm Hg oder kleiner als 25 mm Hg, standhält, wie z. B. in der genannten EP 3 068 314 B1 ausgeführt ist.

Wie die Fig. 2A in Draufsicht gezeigte Abschnürvorrichtung 1 erkennen lässt, ragt der von der Vorderseite unter stetiger konvexer Krümmung nach oben hinten gewölbte Betätigungsabschnitt 42 der Wippe 4 in eine zur Längsrichtung des durchgeführten Abschnürbands 3 und zur Längsachse des Schlossgehäuses 2 symmetrische Aussparung, wobei im unbetätigten Ruhezustand der Wippe 4 die Oberseite des Betätigungsabschnitts 42 und der Deckwand 24 des Schlossgehäuses 2 über einen Zwischenspalt geringer, aber fühlbarer Breite im Wesentlichen flächenbündig ineinander übergehen. Entsprechend geht auch der im hinteren Abschnitt des Schlossgehäuses 2 verrastend eingesetzte Rastschuh 5 in seinem zur Vorderseite hin gelegenen Randbereich im Wesentlichen flächenbündig über eine angepasste, zur Längsachse symmetrische Ausnehmung in die Oberseite der Deckwand 24 über. Durch diese Ausgestaltung ergibt sich eine vorteilhafte Bedienbarkeit der Abschnürvorrichtung 1 beim dosierten, spannungsgerechten Festlegen der Abschnürvorrichtung 1 an einem abzuschnürenden Körperteil bzw. beim Lösen und/oder Freigeben der Abschnürvorrichtung 1.

Benutzungsvorteile für die Bedienung bzw. Handhabung und Aufbewahrung ergeben sich auch aus der Anbringung des am vorderen Endbereich des Abschnürbands 3 angebrachten Abschlussstücks 6. Dieses dient z. B. als Anschlag, um ein unbeabsichtigtes Herausziehen des Abschnürbands 3 aus dem Schlossgehäuse 2 zu vermeiden und ist beispielsweise, wie aus Fig. 2A ersichtlich, mit einer Klemmzunge und/oder einer Halteöffnung als Halterung zum Aufbewahren versehen. Die Anbringung des Abschlussstücks 6 an dem Abschnürband 3 erfolgt z. B. über eine rückseitig an dem Abschlussstück 6 ausgebildete Klemmaufnahme (s. Fig. 2B) und/oder allein oder zusätzlich über eine stoffschlüssige Verbindung wie Kleben oder Schweißen.

Wie Fig. 2B und in einem vergrößerten Ausschnitt Z die Fig. 2C weiter zeigen, ist das Abschnürband, wie vorstehend beschrieben, unter Bildung der Schlaufe 31 von der Rückseite her in den im Innern des Schlossgehäuses 2 gebildeten Durchführschacht 230 zwischen der Oberseite der Wippenplatte 41 und der Unterseite einer in dem Schlossgehäuse 2 angeformten Zwischenwand 25 geführt und auf der Vorderseite durch eine Durchführöffnung 43 im Übergangsbereich zwischen der Vorderseite der Wippenplatte 41 und dem Betätigungsabschnitt 42 der Wippe 4 herausgeführt. Die Zwischenwand 25 ist auf ihrer Unterseite in ihrem vor der Schwenklagerung liegenden vorderen Abschnitt, der sich bis auf einen geringen Spalt für die Betätigung der Wippe 4 bis zur Innenseite des Betätigungsabschnitts 42 erstreckt, mit einer Haltestruktur in Form einer quer (vorzugsweise rechtwinklig) zur Längserstreckung des Schlossgehäuses 2 verlaufenden vorderen Unterkante 241 versehen, die zur Oberseite 32 des Abschnürbands 3 hin vorsteht und zur Rückseite hin eine Auflaufschräge und zur Vorderseite hin eine steile Halteflanke bildet, so dass das elastische gummiartige Abschnürband beim Andrücken gegen die Zwischenwand unter Verschwenken der Wippe 4 durch Zug nach unten an dem rückseitig eingeführten Schlaufenabschnitt 3 in Folge der eindringenden Unterkante 251 mittels der steilen Vorderflanke gegen ein Zurückziehen festgelegt ist. Durch Druck auf den Betätigungsabschnitt 42 kann der Benutzer die Fixierung bzw. Festklemmkraft des Abschnürbands 3 dosiert lösen. Die Zwischenwand 25 mit der genannten Haltestruktur in Form der vorderen Unterkante 251 bildet somit ein Widerlager zum Festklemmen des Abschnürbands 3 mittels der Wippe 4.

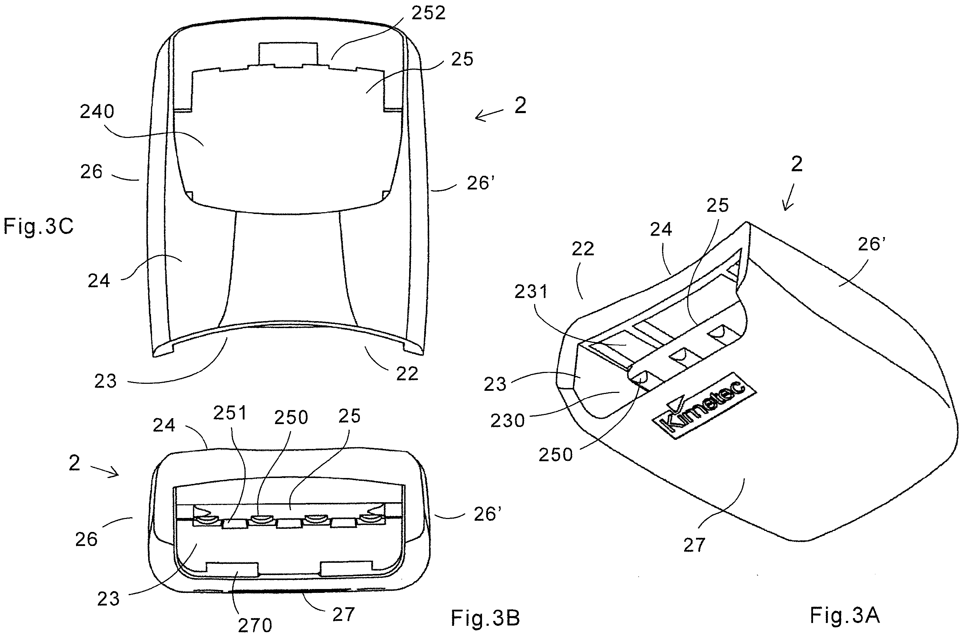

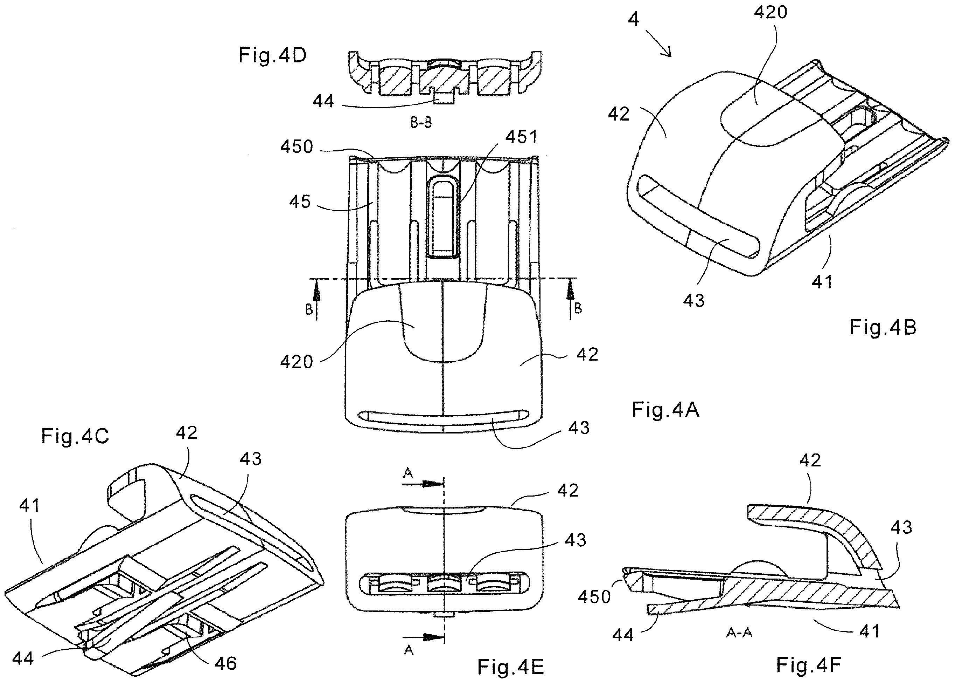

Wie die Fig. 2B und insbesondere 2C weiter zeigen, ist bei diesem Ausführungsbeispiel die Schwenklagerung etwa mittig zwischen der Vorderseite und der Rückseite des Schlossgehäuses 2 im Bereich der Unterseite der Wippenplatte 41 ebenfalls etwa mittig bezüglich dieser ausgebildet. Die die Schwenklagerung bildenden Elemente auf der Bodenseite des Schlossgehäuses 2 und der Unterseite der Wippenplatte 41 werden nachfolgend anhand der Fig. 3A bis 3C und 4A bis 4F noch näher beschrieben.

Wie die Fig. 2B und 2C noch weiter zeigen, ist die Wippenplatte 41 in ihrem hinteren unteren Bereich mit einer Federanordnung in Form einer Rückstellfeder 44 versehen, die vorzugsweise einstückig an der Wippenplatte 41 im Bereich hinter der Schwenklagerung angeformt ist und schräg nach hinten unten bis nahe an das hintere Ende der Wippenplatte 41 erstreckt ist. Die so zungenartig gestaltete Rückstellfeder 44 stützt sich an ihrem hinteren Endbereich mit ihrer Unterseite auf der Oberseite der Bodenwand 27 des Schlossgehäuses 2 ab, wobei der Abstand zwischen der Unterseite der Rückstellfeder 44 und der Oberseite der Wippenplatte 41 so dimensioniert ist, dass die Wippenplatte 41 in dem unbelasteten Zustand des Abschnürbands 3 (d. h. ohne nach unten gerichteten Zug auf den von der Rückseite her eingeführten Bandabschnitt der Schlaufe 31) zumindest weitgehend parallel zur Unterseite der Zwischenwand 25 ausgerichtet ist, so dass das Abschnürband 3 in jedem Falle ohne Ausübung einer Klemmkraft durch die Wippe 4 leicht in dem Durchführschacht 230 verschiebbar und damit z. B. die Schlaufenweite weitgehend ohne Kraftaufwand variierbar ist, bevor ein dosiertes Festlegen an dem abzuschnürenden Körperteil erfolgt. Auch nach Öffnen der Schlaufe 31 durch Entrasten des Rastschuhs 5 ist das Abschnürband 3 somit leicht in dem Durchführschacht 230 verschiebbar. Das leichte Verschieben des Abschnürbands 3 in dem Schlossgehäuse 2 hängt dabei wesentlich auch von den guten Gleiteigenschaften des Abschnürbands 3 in dem (i. d. R. aus härterem Kunststoff bestehenden) Schlossgehäuse 2 ab. Der Durchführschacht 230 kann dabei relativ schmal, mit lediglich geringem Spiel zur Querschnittkontur des Abschnürbands 3 ausgelegt werden, so dass sich ein exaktes Ansprechen zum Festklemmen beim Abschnüren und zum Lösen beim Lockern der Schlaufe ergibt.

Ferner ist aus den Fig. 2B und 2C die Einführung und Verrastung des Rastschuhs 5 in dem Schlossgehäuse 2 sowie eine Festlegung des hinteren Endes des Abschnürbands 3 in dem Rastschuh 5 ersichtlich. Demnach ist bei dem gezeigten Ausführungsbeispiel der Rastschuh 5 mit einem Steckabschnitt 51 in einen zwischen der Oberseite der Zwischenwand 25 und der Unterseite der Deckwand 24 gebildeten Aufnahmeschacht 231 durch eine rückseitige Einführöffnung 23 auf der Rückseite 22 des Schlossgehäuses 2 (vgl. auch Fig. 3A, 3B und 3C) eingesteckt und darin mittels einer an ihm nach oben in seinem rückseitigen Bereich vorstehenden Rastnase unter Anlage an einer von der Innenseite der Deckwand 24 nach unten abstehenden Rastgegennase verrastet. Dabei ist der Rastschuh 5 mit einem vorderen Rand eines nach hinten abstehenden Betätigungsteils 51 an einem hinteren Rand der Deckwand 24 des Schlossgehäuses 2 gegebenenfalls mit geringem Spiel in Anschlag gebracht.

Das hintere Ende des Abschnürbands 3 ist in einen Einführschlitz in dem Betätigungsteil 50 über einen auf der Vorderseite desselben nach unten offenen Durchgang eingeführt und in dem Einführschlitz eingeklemmt und kann zusätzlich stoffschlüssig, beispielsweise durch Kleben oder Schweißen, darin festgelegt sein. Das aus dem Durchgang nach unten aus dem Betätigungsteil 50 herausgeführte Abschnürband 3 bildet den hinteren Abschnitt der Schlaufe 31, die im weiteren Verlauf in den vorderen Abschnitt der Schlaufe und anschließend in den in vorstehend beschriebener Weise durch das Schlossgehäuse 2 geführten Abschnitt übergeht. Durch relativ leichten Druck auf die vorteilhaft mit einer leichten Ausmuldung versehene Oberseite des Betätigungsteils 50 kann der Rastschuh 5 einfach entrastet und aus dem Schlossgehäuse 2 herausgezogen werden, um die Schlaufe 31 zu öffnen.

Die Fig. 3A, 3B und 3C zeigen das Schlossgehäuse 2 mit näheren Einzelheiten. Das vorzugsweise aus einem griffigen Kunststoffmaterial mit guter Haptik für die Bedienung ausgebildete Schlossgehäuse 2 ist in seiner Größe zur Aufnahme in einer Hand der Bedienperson und zum leichten Anlegen an einem Körperteil ausgestaltet und weist außen in seinen Übergangsbereichen zwischen den Seitenwänden zur Vorderseite, zur Oberseite und zur Unterseite sanft verlaufende Rundungen für eine gute Handhabung auf. Die Aussparung 240 im vorderen Bereich der Deckwand 24, in die der konvex nach oben hinten verlaufende Betätigungsabschnitt 42 der Wippe 2 hineinragt, ist entsprechend dem Konturverlauf des oberen Rands des Betätigungsabschnitts 42 geformt und symmetrisch zu der vertikalen Mittelängsebene des Schlossgehäuses 2 geformt, wobei durch einen gerundeten Konturverlauf abrupte Kanten vermieden sind. Auf der Rückseite 22 ist der rückseitige Rand der Deckwand 24 und der anschließenden Seitenwände 26, 26' als leichte nach vorne konkave, zur vertikalen Mittelängsebene des Schlossgehäuses 2 symmetrische Einwölbung geformt und an den Konturverlauf des vorderen Rands des Betätigungsteils 50 des Rastschuhs 5 angepasst. Durch die konkaven Einwölbungen der vorderen Aussparung 240 und der hinteren Ausnehmung der Deckwand 24 ergibt sich eine gute Führung für den Einsatz des Betätigungsabschnitts 42 und auch des Rastschuhs 5.

Die vorliegend den Aufnahmeschacht 231 nach unten und den Durchführschacht 230 nach oben begrenzende Zwischenwand 25 ist auf ihrer Unterseite mit in Längsrichtung des Schlossgehäuses 2 verlaufenden, nach unten im Querschnitt (rechtwinklig zur Längsachse) gerundeten, angeformten Gleitrippen 250 versehen, die reibungsmindernd mit der Bandoberseite 32 des Abschnürbands 3 zusammenwirken, so dass dieses im nicht eingeklemmten Zustand leicht, praktisch ohne Hemmung, im Durchführschacht 230 in gewünschtem Ausmaß verschoben werden kann. In Querrichtung zwischen den Gleitrippen 250 sind, wie Fig. 3B erkennen lässt, insbesondere im vorderen Bereich an der Unterseite der Zwischenwand 25 Abschnitte der Haltestruktur in Form der vorderen Unterkante 251 gebildet, die bei Zug nach unten an dem in den Durchführschacht von der Schlaufe 31 her einlaufenden Abschnitt und somit Verschwenken der Wippe 4 mit ihrem vor der Schwenklagerung liegenden vorderen Abschnitt nach oben das Abschnürband 3 gegen die Unterseite der Zwischenwand 45 einklemmt und fixiert. Fig. 3C zeigt auch den Vorderrand 252 der Zwischenwand 25, an dem auch die Gleitrippen und Haltestruktur auslaufen, im Ausführungsbeispiel als in Draufsicht gezahnter Vorderrand 252 mit in Längsrichtung versetzten, in das elastische Abschnürband zum Fixieren eingreifenden Haltestrukturen (Vorsprünge, Rippen oder dgl.).

Wie die Fig. 3B in Ansicht auf die offene Rückseite 22 des Schlossgehäuses 2 mit der rückseitigen Einführöffnung 23 in den Aufnahmeschacht 231 und den Durchführschacht 230 zeigt, ist die Schwenklagerung auf der Oberseite der Bodenwand 27 mittels in Querrichtung rechtwinklig zur Längsachse des Schlossgehäuses 2 verlaufenden Abschnitten einer rippenartigen Schwenkstütze 270 gebildet, auf deren Vorderseite und Rückseite komplementäre Schwenklagerabschnitte an der Unterseite der Wippenplatte 41 bei eingesetzer Wippe 4 positioniert sind. Die Höhe der Schwenkstütze 270 bis zur Unterseite der Wippenplatte 41 zwischen den komplementären Schwenklagerabschnitten ist dabei so gewählt, dass in der Ruhelage der Wippe 4 die lichte Weite des Durchführschachts 230 zwischen der Oberseite der Wippenplatte 41 und der Unterseite der Zwischenwand 25 in Abstimmung auf die Dicke des Abschnürbands 3 ein praktisch ungehemmtes Durchziehen des Abschnürbands 3 ermöglicht und andererseits der Schwenkwinkel so groß ist, dass das Abschnürband 3 durch Verschwenken der Wippe 4 sicher zwischen der Oberseite des vorderen Abschnitts der Wippe 4 und der zugekehrten Unterseite der Zwischenwand 25 gegen die Haltestruktur festgeklemmt wird. Denkbar ist auch z. B. eine Schwenklagerung der Wippe 4 mit seitlich z. B. an der Innenseite der beiden Seitenwände 26, 26' angeordneten Lagerteilen, in die an der Wippe 4 angebrachte komplementäre Lagerteile eingreifen.

Die Fig. 4A, 4B, 4C, 4D, 4E und 4F zeigen ein Ausführungsbeispiel der Wippe 4 mit näheren Einzelheiten. Dabei ist im vorderen Bereich der Wippenplatte 41 der unter konvexer Wölbung nach oben hinten verlaufende Betätigungsabschnitt 42 angeformt, wobei im vorne liegenden Übergangsabschnitt zwischen der Wippenplatte 41 und dem Betätigungsabschnitt 42 die an den Querschnitt des Abschnürbands 3 zum einfachen Durchführen angepasste Durchführöffnung 43 eingebracht ist, wie insbesondere die Fig. 4A, 4B, 4C und 4F zeigen. Wie in Fig. 4B dargestellt, ist auf der Oberseite des nach hinten geführten Teils des Betätigungsabschnitts 42 eine leichte, von der Bedienperson gut tastbare Betätigungsmulde 420 eingeformt. An der Unterseite der Wippenplatte 41 vorstehend ist in deren bezüglich der Querrichtung mittleren Bereich hinter den betreffenden Elementen der Schwenklagerung die schräg nach hinten unten abstehende Rückstellfeder 44 in Form einer an der Wippenplatte 41 angeformten Federzunge angeordnet. In Querrichtung rechtwinklig zur Längserstreckung sind die mit den Abschnitten der Schwenkstütze 270 auf der Oberseite der Bodenwand 27 des Schlossgehäuses 2 zusammenwirkenden Teile der Schwenklagerung nach unten vorstehend angeformt, wobei die Abschnitte der Schwenkstütze 240 zwischen vorderen und hinteren Teilabschnitten der komplementären Schwenklagerabschnitte aufgenommen werden. Die hinteren Teilabschnitte sind dabei an zungenartigen Abschnitten ausgebildet, die zum Einschieben der Wippe 4 in das Innere des Schlossgehäuses 2 über Einlaufschrägen nach oben federnd ausgelenkt werden können, wobei sich betreffende Abschnitte an der Oberseite der Wippenplatte 41 gegen die Unterseite der Zwischenwand 25 abstützen. Zum Einschieben der Wippenplatte 41 kann auch die Rückstellfeder 44 genügend weit gegen ihre Vorspannung nach oben ausgelenkt werden. Zum einfachen Einsetzen bzw. Montieren der Wippenplatte 41 in dem Schlossgehäuse 2 sind ferner an der Unterseite der Wippenplatte 41 in Längsrichtung verlaufende Führungsrippen mit Einführschägen ausgebildet, die mit entsprechenden komplementären Führungsstrukturen auf der Innenseite der Bodenwand 27 führend zusammenwirken. Die federnd ausgebildeten hinteren komplementären Schwenklagerabschnitte sind in Fig. 4C als Federzungen 46 dargestellt.

Wie die Fig. 4A in Draufsicht auf die Wippe 4 zeigt, sind auf der Oberseite der Wippenplatte 41 vorteilhaft weitere Gleitrippen 45 angeformt, um ein Reiben der Bandunterseite 33 des Abschnürbands 3 möglichst gering zu halten. Die Rückstellfeder 44 ist in einer entsprechenden Ausnehmung 451 der Wippenplatte 41 angeordnet. Zum Einsetzen und auch zum einfachen Einführen des Abschnürbands 3 ist der hintere Rand 450 der Wippe 4 vorteilhaft abgeschrägt oder abgerundet. Auch die Wippe 4 besteht vorzugsweise insgesamt aus Kunststoffmaterial, so dass sie für das Abschnürband 3 günstige Gleiteigenschaften und auch ein sicheres Festklemmen ermöglicht und insbesondere der Betätigungsabschnitt 42 eine gute Griffigkeit und gute haptische Eigenschaften für die Betätigung durch den Benutzer ergibt.

Vorteilhaft sind das Schlossgehäuse 2, die Wippe 4 und/oder das Abschnürband 3 transparent für UV-Strahlung, insbesondere im UVC-Spektralbereich, ausgebildet, so dass ein Abtöten von schädlichen Keimen auch in mechanisch schlecht zugänglichen Bereichen mittels UV-Strahlung ermöglicht wird.

Das vorstehend beschriebene, aus einem gummielastischen Material, insbesondere als Silikonband hergestellte, beispielsweise mittels einer Fluoridbehandlung an seiner Außenfläche oder durch andere Zusätze oder eine Behandlung mit UV-Strahlung geglättete und/oder gehärtete Abschnürband 3 ergibt in Kombination mit dem beschriebenen Schlossgehäuse 2 und der Wippe 4 wesentliche Gebrauchsvorteile. Es kann aber auch bei anderer Ausgestaltung des Schlossgehäuses 2 und des Klemmmechanismus vorteilhaft verwendet werden.

## Patentansprüche

1. Abschnürvorrichtung für Körperteile, insbesondere Venenstauer, mit einem Schlossgehäuse (2), in dem ein unten von einer Bodenwand (27), seitlich von zwei Seitenwänden (26, 26') und oben von einer Deckwand (24) umgrenzter, in Längsrichtung verlaufender Durchführschacht (230) gebildet ist, und mit einem Abschnürband (3), das mittels eines an seinem einen, dem rückseitigen Ende befestigten Rastschuh (5) lösbar an einem hinteren Abschnitt des Schlossgehäuses (2) verrastet oder verrastbar und mit seinem anderen Ende zum Bilden einer Schlaufe (31) variierbarer Größe von der Rückseite her durch den Durchführschacht (230) geführt oder führbar und entsprechend der Größe eines aufgenommenen Körperteils mittels eines Klemmmittels, insbesondere mittels einer in dem Schlossgehäuse (2) in einer Schwenklagerung schwenkbar gelagerten Wippe (4), festklemmbar ist,
wobei das Abschnürband (3) aus einem gummielastischen Material wie Naturgummi, einem thermoplastischen Elastomer (TPE) oder insbesondere aus Silikon hergestellt ist,
**dadurch gekennzeichnet,**
**dass** die Bandoberseite (32) und/oder die Bandunterseite (33) mit einer reibungsmindernden reliefförmigen Struktur versehen ist, die die Kontaktflächen zwischen dem Abschnürband (3) und den zugewandten Flächenbereichen im Schlossgehäuse (2) vermindert.

2. Abschnürvorrichtung nach Anspruch 1,
**dadurch gekennzeichnet,**
**dass** das Abschnürband (3) eine gehärtete Oberfläche aufweist.

3. Abschnürvorrichtung nach Anspruch 2,
**dadurch gekennzeichnet,**
**dass** die Oberflächenhärtung durch Fluoridbehandlung eines gummielastischen Bands, insbesondere eines Silikonbands, vorgenommen ist, aus dem das Abschnürband hergestellt ist.

4. Abschnürvorrichtung nach einem der vorhergehenden Ansprüche,
**dadurch gekennzeichnet,**
**dass** das Abschnürband (3) rechteckförmigen Querschnitt aufweist, wobei die Breite des Abschnürbands (3) zwischen 10 und 40 mm, vorzugsweise zwischen 20 und 30 mm, und die Dicke des Abschnürbands (3) zwischen 0,8 und 3 mm, vorzugsweise zwischen 1 und 2,5 mm, beträgt.

5. Abschnürvorrichtung nach einem der vorhergehenden Ansprüche,
**dadurch gekennzeichnet,**
**dass** das Abschnürband (3) mit einer sich bei seiner Dehnung ändernden Markierung versehen ist, anhand deren ein einer Abschnürspannung entsprechender Abschnürzustand des Abschnürbands (3) erkennbar ist.

6. Abschnürvorrichtung nach einem der vorhergehenden Ansprüche,
**dadurch gekennzeichnet,**
**dass** das Abschnürband (3) transparent ist, insbesondere auch für Strahlung im UV-Bereich.

7. Abschnürvorrichtung nach einem der vorhergehenden Ansprüche,
**dadurch gekennzeichnet,**
**dass** das Schlossgehäuse (2) transparent für UV-Strahlung, insbesondere UVC-Strahlung, ist.

8. Abschnürvorrichtung nach einem der vorhergehenden Ansprüche,
**dadurch gekennzeichnet,**
**dass** in dem Durchführschacht (230) an mindestens einer der Bandoberseite (32) und/oder der Bandunterseite (33) zugekehrten Seite von Wandabschnitten oder einer gegebenenfalls vorhandenen Wippe (4) vortretende, in Längsrichtung des Schlossgehäuses (2) verlaufende rippenartige Gleitstrukturen angeordnet sind.

9. Abschnürvorrichtung nach einem der vorhergehenden Ansprüche,
**dadurch gekennzeichnet,**
**dass** bei Ausbildung des Klemmteils als Wippe (4) die Wippe (4) auf ihrer Unterseite in ihrem zur Rückseite hin hinter der Schwenklagerung gelegenen rückseitigen Abschnitt gegen die Oberseite der Bodenwand (27) mittels einer Federanordnung abgestützt ist, durch die die Wippe (4) bei unbelasteter Schlaufe (31) in ihre nicht klemmende Ruhelage gebracht ist.

10. Abschnürvorrichtung nach Anspruch 9,
**dadurch gekennzeichnet,**
**dass** die Federanordnung als an der Wippe (4) angeformte, zur Bodenwand (27) hin vorstehende Rückstellfeder (44) ausgebildet ist.

11. Abschnürvorrichtung nach einem der vorhergehenden Ansprüche,
**dadurch gekennzeichnet,**
**dass** das Abschnürband (3) an seinem von dem Rastschuh (5) abgewandten anderen, dem vorderseitigen Ende mit einem als Halterung ausgebildeten Abschlussstück (6) versehen ist.

## Claims

1. Ligature device for body parts, in particular a tourniquet, comprising a closure housing (2) in which a feed-through shaft (230) is formed which extends in the longitudinal direction and is delimited at the bottom by a base wall (27), laterally by two lateral walls (26, 26') and at the top by a top wall (24), and comprising a ligature strap (3) which, by means of a clipping buckle (5) fastened to the one rear end thereof, is clipped or can be clipped detachably to a rear portion of the closure housing (2) and, with the other end thereof, is guided or can be guided from the rear side through the feed-through shaft (230) in order to form a loop (31) of variable size and can be clamped according to the size of a received body part by means of a clamping means, in particular by means of a rocker (4) that is pivotably mounted in a pivot bearing in the closure housing (2), the ligature strap (3) being produced from a rubber-elastic material such as natural rubber, a thermoplastic elastomer (TPE) or in particular from silicone,
**characterized in that**
the upper side (32) and/or the lower side (33) of the strap is provided with a friction-reducing relief-shaped structure which reduces the contact surfaces between the ligature strap (3) and the facing surface regions in the closure housing (2).

2. Ligature device according to claim 1,
**characterized in that**
the ligature strap (3) comprises a hardened surface.

3. Ligature device according to claim 2,
**characterized in that**
the surface hardening is performed by means fluoride treatment of a rubber-elastic strap, in particular a silicone strap, from which the ligature strap is produced.

4. Ligature device according to any of the preceding claims,
**characterized in that**
the ligature strap (3) has a rectangular cross section, the width of the ligature strap (3) being between 10 and 40 mm, preferably between 20 and 30 mm, and the thickness of the ligature strap (3) being between 0.8 and 3 mm, preferably between 1 and 2.5 mm.

5. Ligature device according to any of the preceding claims,
**characterized in that**
the ligature strap (3) is provided with a marking which changes as it stretches and based on which a ligature state of the ligature strap (3) corresponding to a ligature tension can be identified.

6. Ligature device according to any of the preceding claims,
**characterized in that**
the ligature strap (3) is transparent, in particular also to radiation in the UV range.

7. Ligature device according to any of the preceding claims,
**characterized in that**
the closure housing (2) is transparent to UV radiation, in particular UVC radiation.

8. Ligature device according to any of the preceding claims,
**characterized in that**
rib-like sliding structures extending in the longitudinal direction of the closure housing (2), protruding from wall portions or from an optionally present rocker (4), are arranged in the feed-through shaft (230) on at least one side facing the upper side (32) and/or the lower side (33) of the strap,

9. Ligature device according to any of the preceding claims,
**characterized in that**
when the clamping part is designed as a rocker (4), the rocker (4) is supported on its bottom side, in its rear portion located toward the rear side behind the pivot bearing, against the top side of the base wall (27) by means of a spring arrangement, by means of which the rocker (4) is brought into its non-clamping rest position when the loop (31) is unstressed.

10. Ligature device according to claim 9,
**characterized in that**
the spring arrangement is designed as a return spring (44) formed on the rocker (4) and projecting toward the base wall (27).

11. Ligature device according to any of the preceding claims,
**characterized in that**
the ligature strap (3) is provided at the other end thereof, the front end, facing away from the clipping buckle (5), with an end piece (6) designed as a fastener.

## Revendications

1. Dispositif de ligature pour parties du corps, en particulier garrot, comportant un boîtier de serrure (2) dans lequel est formé un bac de passage (230) s'étendant dans la direction longitudinale et délimité en bas par une paroi de fond (27), latéralement par deux parois latérales (26, 26') et en haut par une paroi de recouvrement (24), et comportant une bande de ligature (3) qui, à l'aide d'un sabot encliquetable (5) fixé à son extrémité arrière, est encliquetée ou peut être encliquetée de manière amovible sur une section arrière du boîtier de serrure (2) et qui, par son autre extrémité, est guidée ou peut être guidée à travers le bac de passage (230) à partir de l'arrière pour former une boucle (31) de taille variable et qui peut être serrée en fonction de la taille d'une partie du corps reçue à l'aide d'un moyen de serrage, en particulier à l'aide d'une pièce basculante (4) montée de manière pivotante dans le boîtier de serrure (2) dans un palier pivotant, dans lequel la bande de ligature (3) est fabriquée en un matériau en caoutchouc élastique, comme le caoutchouc naturel, un élastomère thermoplastique (TPE) ou en particulier en silicone,
**caractérisé en ce**
**que** la face supérieure de bande (32) et/ou la face inférieure de bande (33) est pourvue d'une structure en relief réduisant les frottements, laquelle structure réduit les surfaces de contact entre la bande de ligature (3) et les zones de surface tournées vers celle-ci dans le boîtier de serrure (2).

2. Dispositif de ligature selon la revendication 1,
**caractérisé en ce**
**que** la bande de ligature (3) présente une surface durcie.

3. Dispositif de ligature selon la revendication 2,
**caractérisé en ce**
**que** le durcissement de surface est effectué par traitement au fluorure d'une bande en caoutchouc élastique, en particulier d'une bande en silicone, à partir de laquelle la bande de ligature est fabriquée.

4. Dispositif de ligature selon l'une des revendications précédentes,
**caractérisé en ce**
**que** la bande de ligature (3) présente une section transversale rectangulaire, dans lequel la largeur de la bande de ligature (3) est comprise entre 10 et 40 mm, de préférence entre 20 et 30 mm, et l'épaisseur de la bande de ligature (3) est comprise entre 0,8 et 3 mm, de préférence entre 1 et 2,5 mm.

5. Dispositif de ligature selon l'une des revendications précédentes,
**caractérisé en ce**
**que** la bande de ligature (3) est pourvue d'un marquage qui change lors de son allongement, lequel marquage permet de reconnaître un état de ligature de la bande de ligature (3), état correspondant à une tension de ligature.

6. Dispositif de ligature selon l'une des revendications précédentes,
**caractérisé en ce**
**que** la bande de ligature (3) est transparente, en particulier également au rayonnement dans la plage UV.

7. Dispositif de ligature selon l'une des revendications précédentes,
**caractérisé en ce**
**que** le boîtier de serrure (2) est transparent au rayonnement UV, en particulier au rayonnement UVC.

8. Dispositif de ligature selon l'une des revendications précédentes,
**caractérisé en ce**
**que** des structures coulissantes en forme de nervures, s'étendant dans la direction longitudinale du boîtier de serrure (2), sont agencées faisant saillie dans le bac de passage (230) sur au moins une face, tournée vers la face supérieure de bande (32) et/ou la face inférieure de bande (33), de sections de paroi ou d'une pièce basculante (4) éventuellement présente.

9. Dispositif de ligature selon l'une des revendications précédentes,
**caractérisé en ce**
**que**, lorsque la partie de serrage est réalisée sous forme de pièce basculante (4), la pièce basculante (4) s'appuie sur sa face inférieure, dans sa section arrière située derrière le palier pivotant en direction de la face arrière, contre la face supérieure de la paroi de fond (27), à l'aide d'un agencement à ressort par lequel la pièce basculante (4) est amenée dans sa position de repos de non-serrage lorsque la boucle (31) n'est pas chargée.

10. Dispositif de ligature selon la revendication 9,
**caractérisé en ce**
**que** l'agencement à ressort est réalisé sous la forme d'un ressort de rappel (44) formé sur la pièce basculante (4) et faisant saillie vers la paroi de fond (27).

11. Dispositif de ligature selon l'une des revendications précédentes,
**caractérisé en ce**
**que** la bande de ligature (3) est pourvue, à son autre extrémité opposée au sabot encliquetable (5) et située du côté avant, d'un élément de fermeture (6) réalisé sous forme de support.
